# EUROPÄISCHE PATENTSCHRIFT

(11) **EP 2 586 394 B1**
(45) Veröffentlichungstag und Bekanntmachung des Hinweises auf die Patenterteilung: **13.07.2016**
(21) Anmeldenummer: 11187209.9
(22) Anmeldetag: 28.10.2011
(51) Int. Cl.: A61B 17/88, A61B 17/68

(54) **Schädeldeckelfixierungsvorrichtung**
Skullcap attaching device
Dispositif de fixation de la boîte crânienne

(43) Veröffentlichungstag der Anmeldung: 01.05.2013
(73) Patentinhaber: PINA Medizintechnik Vertriebs AG, 8240 Thayngen (CH)
(72) Erfinder: Piotrowski, Carmen, 8200 Schaffhausen (CH)
(74) Vertreter: Dr. Graf & Partner AG

(56) Entgegenhaltungen:
- EP-A1- 1 985 248
- WO-A1-02/09602
- WO-A2-2007/147057
- DE-C1- 19 952 359
- US-A1- 2002 169 455

## Beschreibung

Die Erfindung betrifft eine Schädeldeckelfixierungsvorrichtung gemäss dem Oberbegriff von Anspruch 1.

### Stand der Technik

Die Druckschrift EP 1985 248 A1 offenbart eine Schädeldeckelfixierungsvorrichtung zum Fixieren benachbarter Schädelknochenplatten. Die Gesamtheit der Schädelknochenplatten bildet die Schädelkalotte, im Englischen als cranial flap bezeichnet. Die Druckschrift offenbart eine handbetätigte Schädeldeckelfixierungsvorrichtung umfassend eine Schädeldeckelklemme sowie einen Applikator, wobei die Schädeldeckelklemme mit Hilfe des Applikators am Schädelknochen angeordnet werden kann und danach durch ein Betätigen des Applikators angezogen werden kann, um die Schädeldeckelklemme dadurch an der Schädelkalotte zu befestigen.

Diese an sich sehr bewährte Schädeldeckelfixierungsvorrichtung könnte das Einführen und das Befestigen der Schädeldeckelklemme verbessert werden.

Das Dokument US2002169455 A1 offenbart eine Schädeldeckelfixierungsvorrichtung gemäß dem Oberbegriff von Anspruch 1.

### Darstellung der Erfindung

Aufgabe der Erfindung ist es ein Schädeldeckelfixierungsvorrichtung zu bilden, welche es ermöglicht die Schädeldeckelklemmen auf einfache Weise an Schädelknochenplatten anzuordnen und zu befestigen.

Diese Aufgabe wird gelöst mit einer Schädeldeckelfixierungsvorrichtung aufweisend die Merkmale von Anspruch 1. Die Unteransprüche 2 bis 10 betreffen weitere vorteilhaft ausgestaltete Schädeldeckelfixierungsvorrichtungen.

Die Aufgabe wird gelöst mit einer Schädeldeckelfixierungsvorrichtung umfassend eine Schädeldeckelklemme mit einem ersten und einem zweiten Klemmelement sowie einem Stab, wobei das erste Klemmelement fest mit dem Stab verbunden ist, und wobei sich der Stab vom ersten Klemmelement aus erstreckt und in einem Endabschnitt endet, und wobei das zweite Klemmelement am Stab verschiebbar angeordnet ist, sowie umfassend einen handbetätigbaren Applikator mit einem Halteteil, einem Stossteil sowie einem Verbindungsteil, wobei das Halteteil und das Stossteil über das Verbindungsteil miteinander verbunden sind, wobei der Endabschnitt des Stabes am Halteteil befestigt ist, und wobei das Stossteil in Verlaufsrichtung des Stabes zwischen dem Halteteil und dem zweiten Klemmelement angeordnet ist, und wobei das Verbindungsteil in Verlaufsrichtung des Stabes derart beabstandet G1616EP zum Stab verläuft, dass ein Verschieben des Verbindungsteils zum Stab hin den Abstand zwischen Halteteil und Stossteil vergrössert, um dadurch das zweite Klemmelement über das darauf einwirkende Stossteil in Richtung des ersten Klemmelementes zu verschieben, wobei der Stab einen Querschnitt aufweist, welcher zumindest teilweise eckig ausgestaltet ist, wobei das zweite Klemmelement eine Ausnehmung aufweist, durch welche der Stab verläuft, wobei das zweite Klemmelement ein Führungsteil umfasst, welches die Ausnehmung auf der einen Seite unter Ausbildung einer Führungsfläche begrenzt, wobei das zweite Klemmelement ein Einrastteil aufweist, welches zur Ausnehmung hin ausgerichtet ist und gegenüber der Führungsfläche angeordnet ist, und wobei der Stab an der dem Einrastteil zugewandten Seite eine strukturierte Oberfläche aufweist und an der der Führungsfläche zugewandten Seite eine erste Führungsseite aufweist, sodass der Stab einerseits über die erste Führungsseite an der Führungsfläche anliegt und andererseits das Einrastteil mit der Oberfläche des Stabes in Wirkverbindung ist, wobei die strukturierte Oberfläche eine Mehrzahl von in Verlaufsrichtung des Stabes nacheinander folgenden Zähnen aufweist. Der Stab weist zwei strukturierte Oberflächen mit Zähnen aufweist, wobei die beiden strukturierten Oberflächen um 90 Grad versetzt angeordnet sind, wobei der Stab zwischen den beiden strukturierten Oberflächen eine in Verlaufsrichtung des Stabes verlaufende zweite Führungsseite aufweist, und wobei das zweite Klemmelement eine Führungsnase aufweist, welche derart angeordnet ist, dass diese zur zweiten Führungsseite hin ausgerichtet ist.

Die erfindungsgemässe Schädeldeckelfixierungsvorrichtung weist den Vorteil auf, dass der Stab wesentlich stabilere Eigenschaften aufweist, was es ermöglichst das erste Klemmelement besser an den Schädelknochenplatten anzuordnen. In einer besonders vorteilhaften Ausgestaltung weist der Querschnitt des Stabes mehrere Ecken auf.

Das zweite Klemmelement weist eine Ausnehmung auf, durch welche der Stab verläuft, wobei das zweite Klemmelement ein Führungsteil umfasst, welches die Ausnehmung auf der einen Seite unter Ausbildung einer Führungsfläche begrenzt, wobei das zweite Klemmelement zudem ein Einrastteil aufweist, welches zur Ausnehmung hin ausgerichtet ist und gegenüber der Führungsfläche angeordnet ist, wobei der Stab an der dem Einrastteil zugewandten Seite eine strukturierte Oberfläche aufweist und an der der Führungsfläche zugewandten Seite eine erste Führungsseite aufweist, sodass der Stab einerseits über die erste Führungsseite an der Führungsfläche anliegt und andererseits das Einrastteil mit der Oberfläche des Stabes in Wirkverbindung ist. Diese Ausgestaltung erlaubt eine besonders sichere und zuverlässige Verbindung zwischen dem Stab und dem zweiten Klemmelement. Nach dem Befestigen der Schädeldeckelklemme an den Schädelknochenplatten wird der über die Schädeldeckelklemme vorstehende Stab abgeschnitten, weshalb es von zentraler Bedeutung ist, dass das erste und zweite Klemmelement der am Schädel verbleibenden Schädeldeckelklemme sicher und langfristig zuverlässig miteinander verbunden sind. Die erfindungsgemässe Ausgestaltung weist den weiteren Vorteil auf, dass ein Verkippen oder Verschwenken des zweiten Klemmelementes bezüglich des Stabes weitgehend oder sogar vollständig verhindert ist. Dies erlaubt es das zweite Klemmelement reproduzierbar und genau dem Stab entlang in Richtung zum ersten Klemmelement hin zu verschieben.

Die strukturierte Oberfläche ist vorzugsweise zahnförmig ausgestaltet, wobei die strukturierte Oberfläche vorteilhafterweise entlang einer ebenen Fläche verläuft, was einen besonders vorteilhaften Eingriff des Einrastteils in die strukturierte Oberfläche ermöglichst, sodass das zweite Klemmelement besonders gut am Stab gehalten ist.

Der Stab weist zwei entlang der Verlaufsrichtung des Stabes verlaufende strukturierte Oberflächen auf, wobei die beiden strukturierten Oberflächen vorteilhafterweise gegenseitig um 90° versetzt angeordnet sind. Vorteilhafterweise umfasst das zweite Klemmelement zwei Einrastteile, welche derart angeordnet sind, dass jeweils ein Einrastteil in je eine der beiden strukturierten Oberflächen eingreift, sodass das zweite Klemmelement besonders gut am Stab gehalten ist.

In einer besonders vorteilhaften Ausgestaltung weist das zweite Klemmelement eine derart hohe Bauhöhe auf, dass das Einrastteil innerhalb dieser Bauhöhe verlaufend angeordnet ist. Diese Ausgestaltung weist den Vorteil auf, dass das Einrastteil während dem Abschneiden des Stabes besonders gut geschützt ist, sodass auch während und nach dem Abschneiden des Stabes gewährleistet ist, dass das Einrastteil nicht verletzt wurde, und das erste und zweite Klemmteil über den Stab sicher und zuverlässig miteinander verbunden sind.

In einer weiteren vorteilhaften Ausgestaltung umfasst auch das Halteteil ein Einrastteil, welche in den Endabschnitt des Stabes einrastet, um den Stab zuverlässig im Endabschnitt zu befestigen.

In einer weiteren vorteilhaften Ausgestaltung umfasst das Halteteil einen Durchlass, dessen Querschnitt im Wesentlichen der Aussenkontur des Stabes entspricht, sodass der Stab am Durchlass nicht seitlich entweichen kann. Dies ermöglicht einerseits ein besonders zuverlässiges Halten des Stabes im Endabschnitt. Zudem ist die Lage des Stab bezüglich dem Halteteil und den mit dem Halteteil verbundenen Applikator genau definiert, was den Vorteil ergibt, das das sich am Ende des Stabes befindliche erste Klemmelement besonders genau und auch einfach positioniert werden kann.

Der Applikator ist derart ausgestaltet, dass ein Betätigen eines Betätigungsabschnittes quer zur Verlaufsrichtung des Stabes erfolgt, vorzugsweise im Wesentlichen in zur Verlaufsrichtung des Stabes senkrechter Richtung. In einer bevorzugten Ausführungsform wird die Schädeldeckelfixierungsvorrichtung während dem Einführen der Schädeldeckelklemme in den Schädelknochen auch am Betätigungsabschnitt gehalten. Dies ergibt den Vorteil, dass die Schädeldeckelfixierungsvorrichtung mit einer einzigen Hand sowohl im Schädelknochen positioniert werden kann, und danach durch ein Drücken auf den Betätigungsabschnitt die Schädeldeckelklemme angezogen beziehungsweise fixiert werden kann. Dadurch dass der Betätigungsabschnitt quer zur Verlaufsrichtung des Stabes betätigt wird ergibt sich der Vorteil, dass die Schädeldeckelklemme sehr angenehm und präzise positionierbar und fixierbar ist, indem in einem ersten Handlungsschritt die Schädeldeckelklemme positioniert wird, und dass in einem zweiten Handlungsschritt die Schädeldeckelklemme fixiert wird, wobei währen dem Fixieren eine quer zur Stab verlaufende Kraft auf den Betätigungsabschnitt ausgeübt wird, was zur Folge hat, dass die Schädeldeckelklemme auch während dem Fixieren relativ genau in ihrer Lage gehalten werden kann, sodass das Positionieren und Fixieren der Schädeldeckelklemme unter Verwendung einer einzigen Hand durchgeführt werden kann. Die erfindungsgemässe Schädeldeckelfixierungsvorrichtung weist somit den Vorteil auf, dass diese auf eine für einen Chirurgen sehr angenehme, sichere und schnelle Weise an der Schädelkalotte fixiert werden kann. Nach dem Anziehen beziehungsweise Fixieren der Schädeldeckelklemme muss nur noch der Stab durchtrennt werden, sodass die Schädeldeckelklemme endgültig angeordnet ist und die nächste Schädeldeckelklemme gesetzt werden kann.

In einer bevorzugten Ausgestaltung ist die Schädeldeckelfixierungsvorrichtung als Einwegteil ausgestaltet, indem diese jeweils einen handbetätigbaren Applikator und eine damit verbundene Schädeldeckelklemme umfasst, wobei der handbetätigbare Applikator nach dem Positionieren und Fixieren der Schädeldeckelklemme entsorgt wird. Diese Ausgestaltung weist den Vorteil auf, dass ein Chirurge eine Mehrzahl von Schädeldeckelklemmen in kurzer Zeit nacheinander an der Schädelkalotte befestigen kann.

In einer weiteren Ausgestaltung kann der handbetätigbare Applikator auch mehrfach benutzt werden, wobei der Applikator einen Befestigungsabschnitt aufweist, an welchem jeweils eine Schädeldeckelklemme befestigbar ist, um diese in die Schädelkalotte einzuführen und mit der Schädelkalotte zu befestigen. Ein Vorteil dieser Ausgestaltung ist darin zu sehen, dass einem Chirurgen ein Set zur Verfügung gestellt werden kann, umfassend einen Applikator und eine Mehrzahl von Schädeldeckelklemmen, falls erforderlich auch unterschiedlich ausgestaltete Schädeldeckelklemmen. Ein weitere Vorteil dieser Ausgestaltung ist darin zu sehen, dass auch Schädeldeckelklemmen anderer Hersteller mit dem handbetätigbaren Applikator in die Schädelkalotte eingeführt und mit der Schädelkalotte befestigt werden können.

Nachfolgend werden mehrere Ausführungsbeispiele im Detail erläutert.

### Kurze Beschreibung der Zeichnungen

Die zur Erläuterung der Ausführungsbeispiele verwendeten Zeichnungen zeigen:
- Fig. 1: eine perspektivische Ansicht eine Schädeldeckelfixierungsvorrichtung;
- Fig. 2: eine Draufsicht der in Figur 1 dargestellten Schädeldeckelfixierungsvorrichtung;
- Fig. 3: eine Seitenansicht der in Figur 1 dargestellten Schädeldeckelfixierungsvorrichtung;
- Fig. 4: eine perspektivische Ansicht eines Applikators;
- Fig. 5: eine Seitenansicht einer Schädeldeckelklemme, wobei das eine Klemmelement im Schnitt dargestellt ist;
- Fig. 6: eine Befestigungsvorrichtung des Applikators im Detail;
- Fig. 7: eine Ansicht des Applikators entlang des Schnittes A-A;
- Fig. 8: eine Schädeldeckelfixierungsvorrichtung welche betätigt wurde, sodass die Schädeldeckelklemme zusammengeführt ist;
- Fig. 9: eine Draufsicht auf ein zweites Klemmelement;
- Fig. 10: einen Querschnitt durch einen Stab;
- Fig. 11: eine Detailansicht einer strukturierten Oberfläche eines Stabes;
- Fig. 12: eine zusammengeführte Schädeldeckelklemme in einer Seitenansicht wobei das zweite Element im Schnitt dargestellt ist;
- Fig. 13: die in Figur 12 dargestellte Schädeldeckelklemme in perspektivischer Ansicht;
- Fig. 14: die in Figur 12 dargestellte Schädeldeckelklemme 2 nach dem Durchtrennen des Stabes;
- Fig. 15: eine Draufsicht auf ein weiteres Ausführungsbeispiel eines zweiten Klemmelementes;
- Fig. 16: einen Schnitt durch ein weiteres Ausführungsbeispiel eines Stabes;
- Fig. 17: einen Schnitt durch ein weiteres Ausführungsbeispiel eines Stabes sowie schematisch ein zweites Klemmelement;
- Fig. 18: Schnitte durch zwei weitere Ausführungsbeispiele eines Stabes.

Grundsätzlich sind in den Zeichnungen gleiche Teile mit gleichen Bezugszeichen versehen.

### Wege zur Ausführung der Erfindung

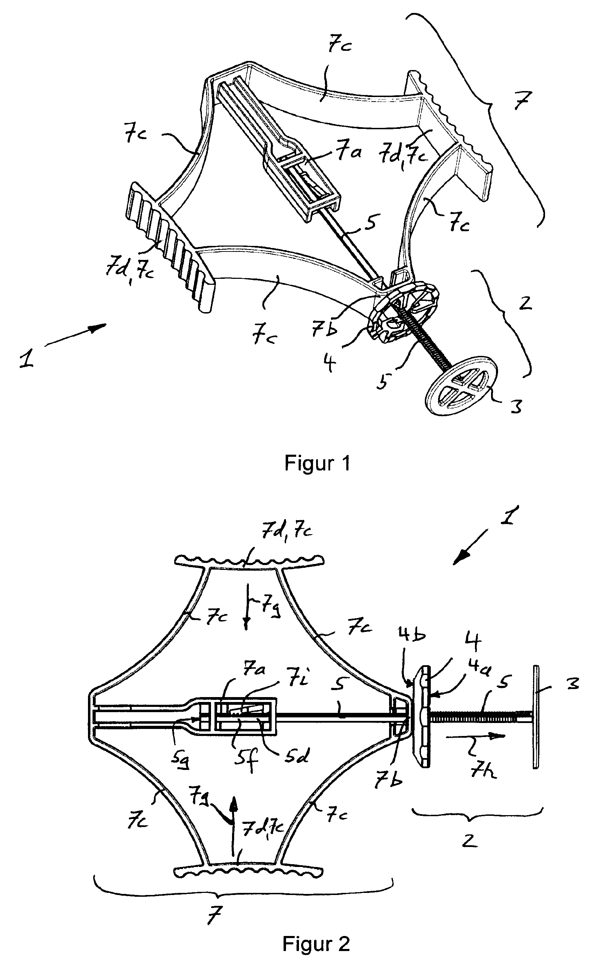

Figur 1 zeigt in einer perspektivischen Ansicht eine Schädeldeckelfixierungsvorrichtung 1, bestehend aus einer Schädeldeckelklemme 2 und einem handbetätigbaren Applikator 7. In einer bevorzugten Ausgestaltung sind der Applikator 7 und die Schädeldeckelklemme 2 als separate Teile ausgestaltet, welche nach deren Herstellung miteinander zu der Schädeldeckelfixierungsvorrichtung 1 verbunden werden. Die Schädeldeckelklemme 2 umfasst ein erstes Klemmelement 3, ein zweites Klemmelement 4 sowie einen Stab 5. Das erste Klemmelement 3 ist fest mit dem Stab 5 verbunden, und das zweite Klemmelement 4 ist in Verlaufsrichtung des Stabes 5 auf dem Stab 5 zum ersten Klemmelement 3 hin verschiebbar gelagert. Der Applikator 7 umfasst ein Stossteil 7b sowie ein Halteteil 7a, wobei das Stossteil 7b und das Halteteil 7a über das flexible Verbindungsteil 7c miteinander verbunden sind. In einer vorteilhaften Ausgestaltung sind die im wesentlichen parallel zum Stab 5 verlaufenden Teile des Verbindungsteils 7c als Betätigungsabschnitt 7d beziehungsweise als An- oder Auflageflächen für die Finger ausgestaltet. In einer besonders vorteilhaften Ausgestaltung stehen die Betätigungsabschnitt 7d, wie in Figur 1 dargestellt, ein oder beidseitig über die nachfolgenden Verbindungsteil 7c vor, sodass sich in Verlaufsrichtung des Stabes 5 ein besonders langer Betätigungsabschnitt 7d ergibt, was den Vorteil aufweist, dass die An- oder Auflagefläche für die Finger besonders lang und zum Halten angenehm ausgestaltet ist. In einer besonders vorteilhaften Ausgestaltung weist der Betätigungsabschnitt 7d, wie in Figur 1 dargestellt, eine Oberflächenstruktur wie zum Beispiel Rippen auf, um den Betätigungsabschnitt 7d mit den Fingern, und insbesondere mit Fingern, bei welchen ein Handschuh überzogen ist, besonders gut zu halten. Ein derartiger Betätigungsabschnitt 7d erlaubt es den Applikator 7 besonders gut zu halten, das erste Klemmelement 3 besonders gut in die Schädelplatten einzuführen, und die Betätigungsabschnitt 7d besonders gut zusammen zu drücken, um dadurch die Schädeldeckelklemme 2 zu befestigen.

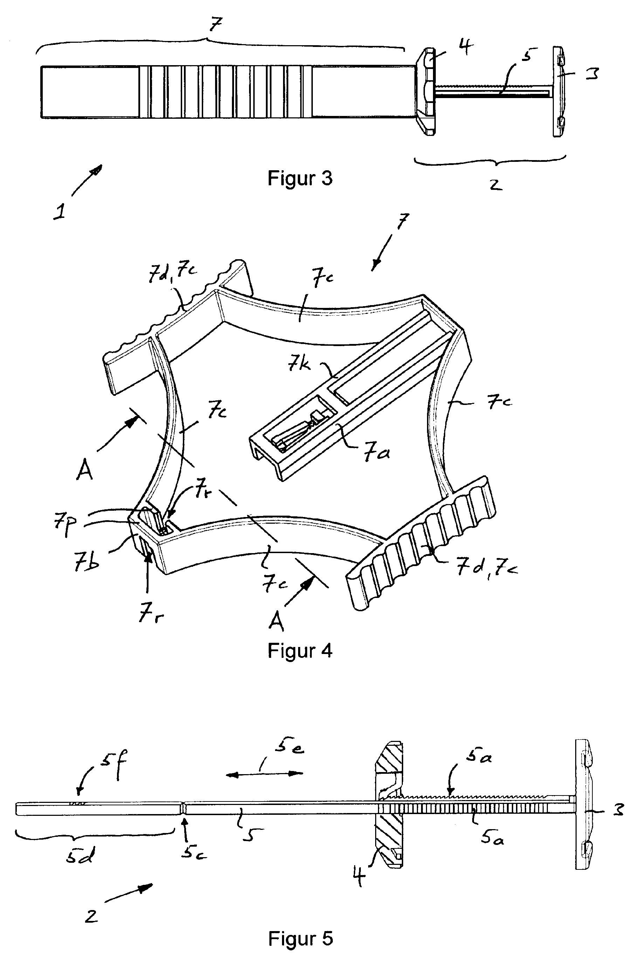

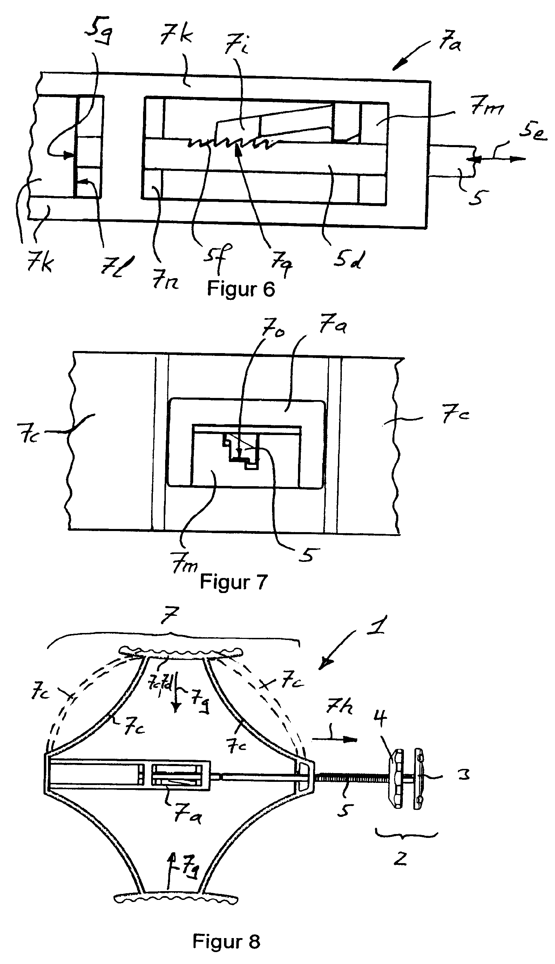

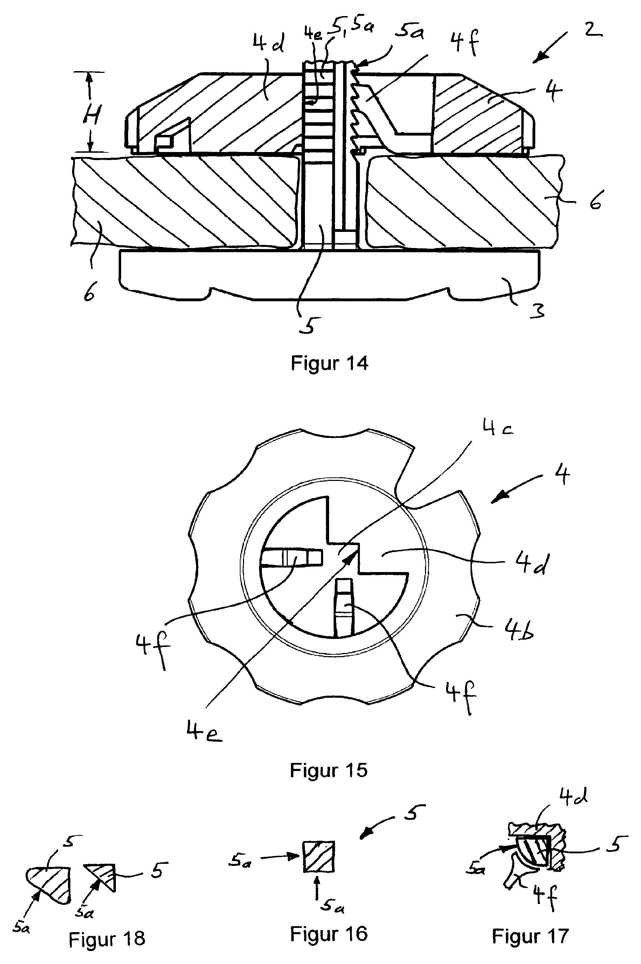

Figur 2 zeigt eine Draufsicht auf die Schädeldeckelfixierungsvorrichtung 1 gemäss Figur 1, und Figur 3 zeigt eine Seitenansicht der Schädeldeckelfixierungsvorrichtung 1. Wie aus den Figuren 1 und 2 ersichtlich, ist die Schädeldeckelklemme 2 derart im Applikator 7 angeordnet, dass der Stab 5 mit einem Endabschnitt 5d in dem Halteteil 7a festgehalten ist. In einer bevorzugten Ausgestaltung umfasst das Halteteil 7a, wie in Figur 2 dargestellt, ein Einrastteil 7i, welches in die Oberfläche des Endabschnittes 5d einrastet und ein Verschieben des Stabes 5 bezüglich des Halteteils 7a zu verhindern. Der Applikator 7 umfasst zudem ein Stossteil 7b, welches am zweiten Klemmelement 4 anliegt. Das flexible Verbindungsteil 7c des Applikators 7 ist derart wirkend ausgestaltet, dass ein Zusammendrücken der beiden gegenüberliegend angeordneten Betätigungsabschnitte 7d zur Folge hat, dass diese in Bewegungsrichtung 7g, welche senkrecht zur Verlaufsrichtung des Stabes 5 verläuft, zusammengedrückt werden, was zur Folge hat, dass das Stossteil 7b in Bewegungsrichtung 7h bewegt wird, was zur Folge hat, dass das zweite Klemmteil 4 zum ersten Klemmteil 3 hin verschoben wird. Der Applikator 7 kann auf unterschiedlichste Weise verlaufend ausgestaltet sein um diese Funktion zu bewirken. Figur 8 zeigt zum Beispiel strichliert ein weiterer, alternativer Verlauf der flexiblen Verbindungsteile 7c, um diese Funktion zu bewirken. Die Verbindungsteile 7c könnten auch nur einseitig angeordnet sein, die in Figur 8 durch die strichlierten Verbindungsteile 7c dargestellt, wobei der eine Finger dann am Betätigungsabschnitt 7d anliegen würde und der andere Finger am Halteteil 7a. Die erfindungsgemässe Schädeldeckelfixierungsvorrichtung 1 wird zum Beispiel verwendet, um nach einer erfolgten Gehirnoperation eine vor der Operation entfernte Schädelknochenplatte wieder mit der Schädelkalotte zu verbinden und an dieser zu befestigen. Dazu wird die Schädelknochenplatte an der Schädelkalotte angelegt, und das erste Klemmelement zwischen Schädelknochenplatte und Schädelkalotte eingeführt. Bei diesem Vorgang wird der Applikator 7 vorzugsweise an dem Betätigungsabschnitten 7d mit je einem Finger gehalten, sodass durch eine entsprechende Handbewegung die Position des ersten Klemmelementes 3 geführt werden kann und das erste Klemmelement 3 präzise an der Schädelkalotte positioniert werden kann. Die erfindungsgemässe Schädeldeckelfixierungsvorrichtung 1 weist insbesondere den Vorteil auf, dass das erste Klemmelement 3 über den Stab 5 relativ starr mit dem Applikator 7 verbunden ist, was zur Folge hat, dass das erste Klemmelement 3 sehr präzise bezüglich der Schädelkalotte und der Schädelknochenplatte eingeführt beziehungsweise positioniert werden kann. In einem zweiten Verfahrensschritt werden die beiden Betätigungsabschnitte 7d in Bewegungsrichtung 7g zusammengedrückt, was zur Folge hat, dass das zweite Klemmelement 4 zum ersten Klemmelement 3 hin verschoben wird, sodass die sich dazwischen befindliche Schädelknochenplatte bezüglich der Schädelkalotte fixiert wird. In einem nachfolgenden Schritt wird die auf den Betätigungsabschnitt 7d bewirkte Kraft reduziert, sodass das Stossteil 7b wieder zurückbewegt wird, beispielsweise in die in Figur 2 dargestellte Lage, wobei das zweite Klemmelement 4 in der vorher eingenommen Lage verharrt, wie dies in Figur 8 dargestellt ist. In einem nachfolgenden Schritt wird, wie aus Figur 8 ersichtlich, der Stab 5 vorzugsweise unmittelbar nach dem zweiten Klemmelement 4 geschnitten, sodass die Schädeldeckelklemme 2, wie beispielsweise in Figur 14 dargestellt, ohne oder mit einem vorzugsweise nur minimal vorstehenden Stab 5 am den Knochenplatten 6 fixiert ist.

Figur 4 zeigt eine perspektivische Ansicht des Applikators 7 ohne eingeführte Schädeldeckelklemme 2. Der Applikator umfasst ein Halteteil 7a, welches über einen Haltearm 7k mit dem flexiblen Verbindungsteil 7c verbunden ist, wobei das flexible Verbindungsteil 7c zudem mit dem Stossteil 7b verbunden ist. In einer bevorzugten Ausgestaltung sind die im Wesentlichen parallel verlaufenden Abschnitte des flexiblen Verbindungsteils 7c als Betätigungsabschnitte 7d ausgestaltet. Vorzugsweise stehen diese auch in Verlaufsrichtung des Stabes 5 vor, sodass ein angenehmes Halten und Betätigen mit den Fingern möglich ist. In einer vorteilhaften Ausgestaltung ist das Stossteil 7b zudem als ein Führungsteil 7p mit Durchlass 7r ausgestaltet, wobei bei eingefügter Schädeldeckelklemme 2 der Stab 5 durch den Durchlass 7r verläuft und über das Führungsteil 7p seitlich zumindest teilweise geführt ist. Der Durchlass 7r ist in Figur 4 u-förmig ausgestaltet. Der Durchlass 7r könnte jedoch auch als Durchbruch ausgestaltet sein, beispielsweise als ein Loch. In einer vorteilhaften Ausgestaltung ist dem Stossteil 7b nachfolgend ein weiteres Führungsteil 7p mit Durchbruch 7r angeordnet, wobei die beiden Führungsteile 7p vorteilhafterweise wie dargestellt derart gegengleich angeordnet sind, dass die Öffnung des einen Durchlasses 7r in die eine Richtung ausgerichtet ist, und die Öffnung des anderen Durchlasses 7r in die entgegen gesetzte Richtung ausgerichtet ist. Ein durch diese beiden Durchlasse 7 verlaufender Stab 5 ist durch die Führungsteile 7p besonders gut geführt und gehalten, was den Vorteil ergibt, dass die Bewegung des Stabes 5 besonders gut an die Bewegung der Betätigungsabschnitte 7d gekoppelt ist.

Figur 5 zeigt eine Seitenansicht der Schädeldeckelklemme 2. Im dargestellten Ausführungsbeispiel weist die Oberfläche des Stabes 5 im Bereich zwischen dem zweiten Klemmelement 4 und erstem Klemmelement 3 eine Verzahnung 5a auf. Der Stab 5 umfasst zudem an dem dem ersten Klemmelement 3 gegenüberliegenden Ende einen Endabschnitt 5d beziehungsweise einen Befestigungsabschnitt mit einer Verzahnung 5f. Der Stab 5 kann in einer vorteilhaften Ausgestaltung zudem eine Dünnstelle 5c umfassen, um eine bessere Beweglichkeit des Befestigungsabschnittes 5d bezüglich dem ersten Klemmelement 3 zu bewirken.

Figur 6 zeigt ein Ausführungsbeispiel eines Halteteils 7a des Applikators 7. In diesem Ausführungsbeispiel ist der Stab 5 wie in Figur 7 in einem Querschnitt dargestellt als mehreckiger Querschnitt ausgestaltet. Der Stab 5 umfasst einen Endabschnitt 5d mit einer Verzahnung 5f. Das Halteteil 7a umfasst ein Einrastteil 7i, dessen Stirnseite 7q eine Verzahnung aufweist, welche gegengleich zur Verzahnung 5f ausgestaltet ist. Das Einrastteil 7i ist zudem senkrecht zur Verlaufsrichtung 5e des Stabes 5 elastisch gelagert und weist eine zum Stab 5 hin ausgerichtete Vorspannkraft aus. Dies hat zur Folge, dass der Endabschnitt 5d der Stab 5 bezüglich der Bewegungsrichtung 5e nach der Einführung des Stabes 5 fest im Halteteil 7a gelagert ist. In einer weiteren bevorzugten Ausgestaltung bildet der Haltearm 7k zudem eine Stossfläche 71 aus, an welcher die Stirnseite 5g des Endabschnittes 5d des Stabes 5 anliegt. Dies ergibt den Vorteil, dass über den Applikator 7 auch eine Presskraft auf den Stab 5 beziehungsweise auf das erste Klemmelement 3 ausgeübt werden kann.

Figur 7 zeigt eine Ansicht des Applikators 7 in einem Schnitt entlang der in Figur 4 dargestellten Schnittlinie A-A. In diesem Ausführungsbeispiel umfasst das Halteteil 7a ein Führungsteil 7m mit einer Ausnehmung bzw. einem Durchlass 7o. Der Durchlass 7o ist bezüglich dem Querschnitt des Stabes 5 vorzugsweise derart angepasst ausgestaltet, dass der Stab 5 seitlich durch den Durchlass 7o geführt ist und zwischen dem Führungsteil 7m und dem Stab 5 kein oder ein nur minimaler Spalt entsteht, sodass der Stab 5 durch dass Führungsteil 7m seitlich geführt ist. Diese Ausgestaltung weist den Vorteil auf, dass der Stab 5 besonders gut und präzise im Halteteil 7a gehalten ist, da der Stab 5 einerseits mit der Stirnseite 5g des Endabschnittes 5d an der Stossfläche 71 anliegt, und andererseits der Stab 5 seitlich am Führungsteil 7m gehalten ist, und der Stab 5 über das Einrastteil 7i in Bewegungsrichtung 5e fest gehalten ist. In einer bevorzugten Ausgestaltung ist, wie in Figur 6 dargestellt, zudem ein weiteres Führungsteil 7n im Halteteil 7a angeordnet, welches beispielsweise identisch ausgestaltet sein kann wie das rechts und auch in Figur 7 dargestellte Führungsteil 7n, um den Stab 5 zusätzlich in Halteteil 7a zu führen, sodass der Endabschnitt 5d trotz der über das Einrastteil 7i einwirkenden Kräfte oder trotz in Verlaufsrichtung 5e einwirkenden Kräfte nicht entweichen kann. Dies bewirkt eine besonders zuverlässige Verbindung des Einrastteils 7i mit der Einraststelle 5f des Stabes 5.

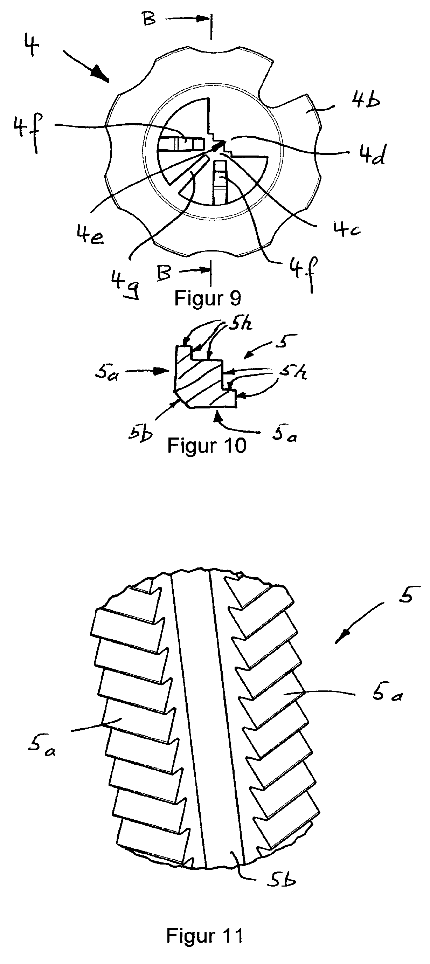

Figur 9 zeigt in einer Draufsicht ein Ausführungsbeispiel eines zweiten Klemmelementes 4, wobei die auch in Figur 2 bezeichnete Aussenfläche 4b dargestellt ist. Das zweite Klemmelement 4 umfasst eine Öffnung 4c und umfasst ein Führungsteil 4d, welches eine senkrecht zur Betrachtungsebene verlaufende Führungsfläche 4e ausbildet. Gegenüberliegend zu dieser Führungsfläche 4e sind zwei Einrastteile 4f angeordnet, welche als Rastnasen ausgestaltet sind. Im dargestellten Ausführungsbeispiel ist zudem noch eine zur Öffnung 4c hin vorstehend Führungsnase 4g angeordnet.

Figur 10 zeigt den Querschnitt eines Stabes 5, welcher zur Aufnahme in das in Figur 9 dargestellte zweite Klemmelement 4 bestimmt ist. Der Stab 5 umfasst zwei erste Führungsseiten 5h, welche, sofern durch das zweite Klemmelement 4 verlaufend, beide an der Führungsfläche 4e des zweiten Klemmelementes 4 anliegen. Der Stab 5 umfasst zudem zwei Flächen mit strukturierter Oberfläche 5a, welche als Verzahnungen ausgestaltet sind, und welche im dargestellten Ausführungsbeispiel gegenseitig senkrecht verlaufen.

Der Stab 5 umfasst zudem eine zweite Führungsseite 5b, an welcher, sobald der Stab 5 in das zweite Klemmelement 4 eingeführt ist, die Führungsnase 4g vorzugsweise anliegt. Die Führungsnase 4g hat vorteilhafterweise zur Folge, dass der Stab 5 gegen die Führungsfläche 4e gedrückt wird, und/oder dass das zweite Klemmelement 4 bezüglich der Öffnung 4c beziehungsweise bezüglich dem Stab 5 präzise und reproduzierbar angeordnet ist. In einer besonders vorteilhaften Ausgestaltung weist das zweite Klemmelement 4, wie in Figur 14 dargestellt, eine Höhe H auf, welche beispielsweise in einem Bereich zwischen 1 und 5 mm liegen kann, was zur Folge hat, dass die Führungsfläche 4e die Länge der Höhe H aufweisen kann, was zur Folge hat, dass ein Kippen des zweiten Klemmelementes 4 bezüglich des Stabes 5 stark reduziert oder völlig verhindert werden kann, insbesondere da der Stab 5 vorzugsweise an der Führungsfläche 4e anliegt aufgrund der auf den Stab 5 wirkenden Führungsnase 4g und/oder der auf den Stab 5 wirkenden Einrastteile 4f.

Figur 11 zeigt in einer perspektivischen Detailansicht den in Figur 10 dargestellten Stab 5, mit einer zweiten Führungsseite 5b und zwei als Verzahnungen ausgestaltete strukturierte Oberflächen 5a.

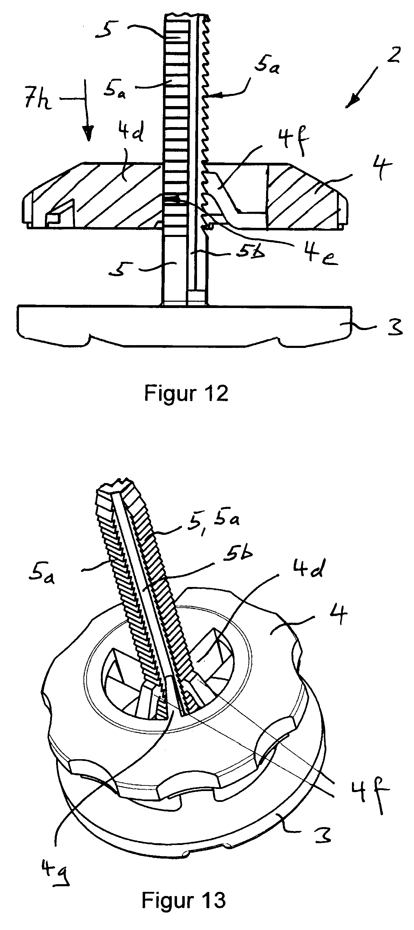

Figur 12 zeigt eine Seitenansicht der Schädeldeckelklemme 2, wobei das zweite Klemmelement 4 im Schnitt dargestellt ist, wobei der Schnitt des Klemmelementes 4 entlang der in Figur 9 dargestellten Linie B-B verläuft. Der Stab 5 ist wie in den Figuren 10 und 11 dargestellt ausgestaltet, wobei das zweite Klemmelement 4 wie in Figur 9 dargestellt ausgestaltet ist und eine Rastnase 4f aufweist, deren Stirnseite in die Verzahnung 5a eingreift, sodass ein Verschieben des zweiten Klemmelementes 4 in Bewegungsrichtung 7h zum ersten Klemmelement 3 hin möglich ist und ein Verschieben in der zur Richtung 7h entgegen gesetzten Richtung nicht möglich ist. Das dargestellte Ausführungsbeispiel des zweiten Klemmelementes 4 mit Führungsteil 4d, Führungsfläche 4e und Rastnase 4f weist den Vorteil auf, dass eine Bewegung des zweiten Klemmelementes 4 entgegengesetzt zur Richtung 7h nicht möglich ist oder nur unter Verwendung von ausserordentlich hohen Kräften, welche die Rastnase 4f zerstören könnten, möglich ist. Somit ist gewährleistet, dass eine fixierte beziehungsweise angezogene Schädeldeckelklemme 2 zuverlässig und langfristig zusammenhält und nicht auseinander fällt.

Figur 13 zeigt die in Figur 12 dargestellte Anordnung in perspektivischer Ansicht. In Figur 13 ist besonders gut sichtbar dargestellt, wie die beiden als Rastnasen ausgestalteten Einrastteile 4f in die Verzahnungen 5a eingreifen, und wie die Führungsnase 4g auf die zweite Führungsseite 5b einwirkt, um ein reproduzierbares und sicheres Gleiten des zweiten Klemmelementes 4 entlang des Stabes 5 zu bewirken, und um ein sicheres Befestigen des zweiten Klemmelementes 4 bezüglich des Stabes 5 zu gewährleisten.

Figur 14 zeigt eine fixierte Schädeldeckelklemme 2, welche an Knochenplatten 6 anliegt und diese zusammenhält. In Unterschied zu der in Figur 12 dargestellten Anordnung ist in der in Figur 14 dargestellten Anordnung der Stab 5 unmittelbar nach dem zweiten Klemmelement 4 geschnitten. In einer besonders vorteilhaften Ausgestaltung ist die Höhe H des zweiten Klemmelementes 4 und das als Rastnase ausgestaltete Einrastteil 4f derart gegenseitig angepasst ausgestaltet, dass das Einrastteil 4f vollständig innerhalb des zweiten Klemmelementes 4, bzw. entlang der Höhe H innerhalb des zweiten Klemmelementes 4 verläuft. Diese Ausgestaltung weist den Vorteil auf, dass das Einrastteil 4f während dem Durchtrennen des Stabes 5, zum Beispiel mit einer Schere oder einer Zange, vor einer mechanischen Einwirkung der Schere oder der Zange geschützt ist, sodass das Einrastteil 4f nicht beschädigt werden kann, und somit eine sichere Verbindung zwischen dem zweiten Klemmelement 4 und dem Stab 5 währen und insbesondere auch nach erfolgten Schneiden gewährleistet ist.

Figur 15 zeigt eine Draufsicht auf ein weiteres Ausführungsbeispiel eines zweiten Klemmelementes 4, welches im Unterschied zu dem in Figur 9 dargestellten Ausführungsbeispiel keine Führungsnase 4g und eine rechteckige Führungsfläche 4e aufweist.

Figur 16 zeigt einen Querschnitt durch einen Stab 5, welche für das in Figur 15 dargestellte, zweite Klemmelement 4 geeignet wäre. Der in Figur 16 dargestellte Stab 5 weist auf zwei Seiten als Verzahnungen ausgestaltete strukturierte Oberflächen 5a auf.

Figur 17 zeigt ein weiteres Ausführungsbeispiel eines Querschnittes eines Stabes 5 mit einem Querschnitt, welche zumindest teilweise eckig ausgestaltet ist. Der Querschnitt umfasst drei Ecken. Dabei ist die eine Seite kreisförmig oder kurvenförmig verlaufend ausgestaltet, deren Oberfläche 5a strukturiert ist und beispielsweise eine wie in Figur 11 dargestellte, jedoch gekrümmt verlaufende Verzahnung aufweist. Das zweite Klemmelement 4 ist nur ansatzweise dargestellt, und umfasst ein Führungsteil 4d mit Führungsfläche 4e und ein gekrümmt verlaufendes Einrastteil 4f. Auch dieses Ausführungsbeispiel weist die Eigenschaft auf, dass der Stab 5 gegen die Führungsfläche 4e des zweiten Klemmelementes 4 gedrückt wird und dadurch das einwirkende Einrastteil 4f sicher gehalten ist. Der Stab 5 könnte über dessen gesamte Länge denselben, wie in Figur 17 dargestellten Querschnitt aufweisen, wobei in diesem Fall das Halteteil 7a diesem Querschnitt derart anzupassen wäre, dass der Endabschnitt 5d sicher im Halteteil 7a gehalten ist. Der Stab 5 könnte jedoch in dessen Verlaufsrichtung auch die Form des Querschnittes verändern, und beispielsweise links von der Dünnstelle 5c, wie in Figur 5 dargestellt, als rechteckiger Querschnitt ausgestaltet sein. Somit wäre zum Beispiel die in den Figuren 4, 6 und 7 dargestellte Ausführungsform des Halteteils 7a geeignet den Stab 5 zu halten.

Die Figur 18 zeigt links ein weiteres Ausführungsbeispiel eines Stabes 5 mit einem Querschnitt, welche zumindest teilweise eckig ausgestaltet ist. Der Querschnitt umfasst eine Ecke. Die Figur 18 zeigt rechts ein weiteres Ausführungsbeispiel eines Stabes 5 mit einem Querschnitt, welche ausschliesslich eckig ausgestaltet ist. Der Querschnitt umfasst drei Ecken. Die beiden in Figur 18 dargestellten Stäbe könnte beispielsweise in dem in Figur 17 schematisch dargestellten zweiten Klemmelement 4 angeordnet sein, wobei der Verlauf des Einrastteils 4f dem Verlauf der strukturierten Oberfläche 5a anzupassen wäre. In der bevorzugten Ausführungsform weist der Querschnitt des Stabes 5 jedoch ausschliesslich Ecken auf, wie dies in den Figuren 7, 10 und 16 sowie in der Figur 18 rechts dargestellt ist. Besonders vorteilhaft weist der Stab 5 den in Figur 10 dargestellten Querschnitt auf, mit einer Mehrzahl von Führungsseiten 5h, was eine besonders präzise Führung des Stabes 5 bezüglich dem zweiten Klemmelement 4 ermöglicht.

## Patentansprüche

1. Schädeldeckelfixierungsvorrichtung (1) umfassend eine Schädeldeckelklemme (2) mit einem ersten und einem zweiten Klemmelement (3,4) sowie einem Stab (5), wobei das erste Klemmelement (3) fest mit dem Stab (5) verbunden ist, und wobei sich der Stab (5) vom ersten Klemmelement (3) aus erstreckt und in einem Endabschnitt (5d) endet, und wobei das zweite Klemmelement (4) am Stab (5) verschiebbar angeordnet ist, sowie umfassend einen handbetätigbaren Applikator (7) mit einem Halteteil (7a), einem Stossteil (7b) sowie einem Verbindungsteil (7c), wobei das Halteteil (7a) und das Stossteil (7b) über das Verbindungsteil (7c) miteinander verbunden sind, wobei der Endabschnitt (5d) des Stabes (5) am Halteteil (7a) befestigt ist, und wobei das Stossteil (7b) in Verlaufsrichtung (5e) des Stabes (5) zwischen dem Halteteil (7a) und dem zweiten Klemmelement (4) angeordnet ist, und wobei das Verbindungsteil (7c) in Verlaufsrichtung (5e) des Stabes (5) derart beabstandet zum Stab (5) verläuft, dass ein Verschieben des Verbindungsteils (7c) zum Stab (5) hin den Abstand zwischen Halteteil (7a) und Stossteil (7b) vergrössert, um dadurch das zweite Klemmelement (4) über das darauf einwirkende Stossteil (7b) in Richtung des ersten Klemmelementes (3) zu verschieben, wobei der Stab (5) einen Querschnitt aufweist, welcher zumindest teilweise eckig ausgestaltet ist, wobei das zweite Klemmelement (4) eine Ausnehmung (4c) aufweist, durch welche der Stab (5) verläuft, wobei das zweite Klemmelement (4) ein Führungsteil (4d) umfasst, welches die Ausnehmung (4c) auf der einen Seite unter Ausbildung einer Führungsfläche (4e) begrenzt, wobei das zweite Klemmelement (4) ein Einrastteil (4f) aufweist, welches zur Ausnehmung (4c) hin ausgerichtet ist und gegenüber der Führungsfläche (4e) angeordnet ist, und wobei der Stab (5) an der dem Einrastteil (4f) zugewandten Seite eine strukturierte Oberfläche (5a) aufweist und an der der Führungsfläche (4e) zugewandten Seite eine erste Führungsseite (5h) aufweist, sodass der Stab (5) einerseits über die erste Führungsseite (5h) an der Führungsfläche (4e) anliegt und andererseits das Einrastteil (4f) mit der Oberfläche (5a) des Stabes (5) in Wirkverbindung ist, wobei die strukturierte Oberfläche (5a) eine Mehrzahl von in Verlaufsrichtung (5e) des Stabes (5) nacheinander folgenden Zähnen aufweist, **dadurch gekennzeichnet, dass** der Stab (5) zwei strukturierte Oberflächen (5a) mit Zähnen aufweist, wobei die beiden strukturierten Oberflächen (5a) um 90 Grad versetzt angeordnet sind, dass der Stab (5) zwischen den beiden strukturierten Oberflächen (5a) eine in Verlaufsrichtung (5e) des Stabes (5) verlaufende zweite Führungsseite (5b) aufweist, und dass das zweite Klemmelement (4) eine Führungsnase (4g) aufweist, welche derart angeordnet ist, dass diese zur zweiten Führungsseite (5b) hin ausgerichtet ist.

2. Vorrichtung nach Anspruch 1, **dadurch gekennzeichnet, dass** die Führungsnase (4g) an der zweiten Führungsseite (5b) anliegt.

3. Vorrichtung nach Anspruch 1 oder 2, **dadurch gekennzeichnet, dass** die strukturierte Oberfläche (5a) mit Zähnen entlang einer ebenen Fläche verläuft.

4. Vorrichtung nach einem der Ansprüche 1 bis 3, **dadurch gekennzeichnet, dass** das zweite Klemmelement (4) in Verlaufsrichtung (5e) des Stabes (5) eine Bauhöhe (H) aufweist, und dass das Einrastteil (4f) innerhalb dieser Bauhöhe (H) verlaufend ausgestaltet ist, und innerhalb dieser Bauhöhe (H) in die strukturierte Oberfläche (5a) des Stabes (5) eingreift.

5. Vorrichtung nach Anspruch 4, **dadurch gekennzeichnet, dass** die Führungsfläche (4e) in Verlaufsrichtung (5e) des Stabes (5) die Bauhöhe (H) aufweist.

6. Vorrichtung nach einem der vorhergehenden Ansprüche, **dadurch gekennzeichnet, dass** der Endabschnitt (5d) des Stabes (5) einen Einrastabschnitt (5f) aufweist, und dass das Halteteil (7a) ein dem Einrastabschnitt (5f) zugewandtes Einrastteil (7i) aufweist, welches derart in den Einrastabschnitt (5f) eingreift, dass der Stab (5) bezüglich dem Halteteil (7a) fixiert ist.

7. Vorrichtung nach Anspruch 6, **dadurch gekennzeichnet, dass** der Einrastabschnitt (5f) eine Mehrzahl von in Verlaufsrichtung (5e) des Stabes (5) nacheinander folgenden Zähnen aufweist, und dass das Einrastteil (7i) quer zur Verlaufsrichtung (5e) federnd ausgestaltet ist und eine Stirnseite (7q) zum Eingriff in die Zähne aufweist.

8. Vorrichtung nach Anspruch 6 oder 7, **dadurch gekennzeichnet, dass** das Halteteil (7a) auf der dem Stossteil (7b) zugewandten Seite ein Führungsteil (7m) mit eine Durchlass (7o) umfasst, wobei der Durchlass (7o) derart ausgestaltet ist, dass der Stab (5) durch den Durchlass (7o) verläuft, und wobei der Durchlass (7o) zumindest teilweise dem Verlauf des Querschnitts des Stabes (5) angepasst ausgestaltet ist, sodass der Stab (5) zumindest teilweise an Durchlass (7o) anliegen kann.

9. Vorrichtung nach einem der Ansprüche 6 bis 8, **dadurch gekennzeichnet, dass** das Halteteil (7a) über einen Haltearm (7k) mit dem flexiblen Verbindungsteil (7c) verbunden ist, und dass der Haltearm (7k) eine zum Stab (5) hin ausgerichtete Stossfläche (71) umfasst, an welcher die Stirnfläche (5g) des Endabschnittes (5d) des Stabes (5) anliegen kann.

10. Vorrichtung nach einem der vorhergehenden Ansprüche, **dadurch gekennzeichnet, dass** der Applikator (7) einstückig ausgestaltet ist.

## Claims

1. Cranial fixation device (1) comprising a cranial clamp (2) with a first and a second clamp element (3, 4) and with a rod (5), wherein the first clamp element (3) is fixedly connected to the rod (5), and wherein the rod (5) extends from the first clamp element (3) and terminates in an end portion (5d), and wherein the second clamp element (4) is arranged displaceably on the rod (5), and also comprising a manually operable applicator (7) with a holding part (7a), a pushing part (7b) and a connecting part (7c), wherein the holding part (7a) and the pushing part (7b) are connected to each other via the connecting part (7c), wherein the end portion (5d) of the rod (5) is secured on the holding part (7a), and wherein the pushing part (7b) is arranged in the direction of extension (5e) of the rod (5) between the holding part (7a) and the second clamp element (4), and wherein the connecting part (7c) extends in the direction of extension (5e) of the rod (5) and is spaced apart from the rod (5) in such a way that a displacement of the connecting part (7c) towards the rod (5) increases the distance between holding part (7a) and pushing part (7b), in order thereby to displace the second clamp element (4), by way of the pushing part (7b) acting thereon, in the direction of the first clamp element (3), wherein the rod (5) has a cross section with an at least partly polygonal shape, wherein the second clamp element (4) has a recess (4c) through which the rod (5) extends, wherein the second clamp element (4) comprises a guide part (4d) which borders the recess (4c) on one side in order to form a guide surface (4e), wherein the second clamp element (4) has a latching part (4f) which is directed towards the recess (4c) and is arranged opposite the guide surface (4e), and wherein the rod (5) has a structured surface (5a) on its side facing towards the latching part (4f) and has a first guiding side (5h) on its side facing towards the guide surface (4e), such that, on the one hand, the rod (5) bears on the guide surface (4e) via the first guiding side (5h) and, on the other hand, the latching part (4f) is operatively connected to the surface (5a) of the rod (5), wherein the structured surface (5a) has a plurality of teeth arranged successively in the direction of extension (5e) of the rod (5), **characterized in that** the rod (5) has two structured surfaces (5a) with teeth, the two structured surfaces (5a) being arranged offset at 90 degrees in relation to each other, **in that** the rod (5), between the two structured surfaces (5a), has a second guiding side (5b) extending in the direction of extension (5e) of the rod (5), and **in that** the second clamp element (4) has a guide lug (4g), which is arranged in such a way that it is oriented towards the second guiding side (5b).

2. Device according to Claim 1, **characterized in that** the guide lug (4g) bears on the second guiding side (5b).

3. Device according to Claim 1 or 2, **characterized in that** the structured surface (5a) with teeth extends along a level surface.

4. Device according to one of Claims 1 to 3, **characterized in that** the second clamp element (4) has an overall height (H) in the direction of extension (5e) of the rod (5), and **in that** the latching part (4f) is configured to extend within this overall height (H) and engages in the structured surface (5a) of the rod (5) within this overall height (H).

5. Device according to Claim 4, **characterized in that** the guide surface (4e) has the overall height (H) in the direction of extension (5e) of the rod (5).

6. Device according to one of the preceding claims, **characterized in that** the end portion (5d) of the rod (5) has a latching portion (5f), and **in that** the holding part (7a) has a latching part (7i) facing towards the latching portion (5f), which latching part (7i) engages in the latching portion (5f) in such a way that the rod (5) is fixed with respect to the holding part (7a).

7. Device according to Claim 6, **characterized in that** the latching portion (5f) has a plurality of teeth arranged successively in the direction of extension (5e) of the rod (5), and **in that** the latching part (7i) is configured to be resilient in a direction transverse to the direction of extension (5e) and has a front face (7q) for engaging in the teeth.

8. Device according to Claim 6 or 7, **characterized in that** the holding part (7a), on the side facing towards the pushing part (7b), has a guide part (7m) with a passage (7o), wherein the passage (7o) is configured in such a way that the rod (5) extends through the passage (7o), and wherein the passage (7o) is configured to at least partially match the extension of the cross section of the rod (5), such that the rod (5) can at least partially bear on the passage (7o).

9. Device according to one of Claims 6 to 8, **characterized in that** the holding part (7a) is connected to the flexible connecting part (7c) via a holding arm (7k), and **in that** the holding arm (7k) comprises a pushing surface (71) which is oriented towards the rod (5) and on which the front face (5g) of the end portion (5d) of the rod (5) can bear.

10. Device according to one of the preceding claims, **characterized in that** the applicator (7) is configured in one piece.

## Revendications

1. Dispositif de fixation de la boîte crânienne (1) comprenant une pince de boîte crânienne (2) avec un premier et un deuxième éléments de serrage (3, 4) ainsi qu'avec une tige (5), dans lequel le premier élément de serrage (3) est assemblé solidairement à la tige (5), et dans lequel la tige (5) s'étend à partir du premier élément de serrage (3) et se termine dans une partie d'extrémité (5d), et dans lequel le deuxième élément de serrage (4) est agencé de façon déplaçable sur la tige (5), et comprenant également un applicateur (7) actionnable à la main avec une pièce de maintien (7a), une pièce de poussée (7b) ainsi qu'une pièce de liaison (7c), dans lequel la pièce de maintien (7a) et la pièce de poussée (7b) sont reliées l'une à l'autre par la pièce de liaison (7c), dans lequel la partie d'extrémité (5d) de la tige (5) est fixée à la pièce de maintien (7a), et dans lequel la pièce de poussée (7b) est agencée dans la direction d'extension (5e) de la tige (5) entre la pièce de maintien (7a) et le deuxième élément de serrage (4), et dans lequel la pièce de liaison (7c) s'étend à distance de la tige (5), dans la direction d'extension (5e) de la tige (5), de telle manière qu'un déplacement de la pièce de liaison (7c) vers la tige (5) augmente la distance entre la pièce de maintien (7a) et la pièce de poussée (7b), afin de déplacer ainsi le deuxième élément de serrage (4) en direction du premier élément de serrage (3) au moyen de d'élément de poussée (7b) agissant sur lui, dans lequel la tige (5) présente une section transversale qui est au moins partiellement de forme polygonale, dans lequel le deuxième élément de serrage (4) présente un évidement (4c), à travers lequel la tige (5) s'étend, dans lequel le deuxième élément de serrage (4) comprend une pièce de guidage (4d), qui limite l'évidement (4c) sur un côté avec formation d'une face de guidage (4e), dans lequel le deuxième élément de serrage (4) présente une pièce d'encliquetage (4f), qui est dirigée vers l'évidement (4c) et qui est disposée en face de la face de guidage (4e), et dans lequel la tige (5) présente une surface structurée (5a) sur le côté tourné vers la pièce d'encliquetage (4f) et présente un premier côté de guidage (5h) sur le côté tourné vers la face de guidage (4e), de telle manière que d'une part la tige (5) repose par le premier côté de guidage (5h) sur la face de guidage (4e) et que d'autre part la pièce d'encliquetage (4f) soit en liaison active avec la surface (5a) de la tige (5), dans lequel la surface structurée (5a) présente une multiplicité de dents se succédant dans la direction d'extension (5e) de la tige (5), **caractérisé en ce que** la tige (5) présente deux surfaces structurées (5a) avec des dents, dans lequel les deux surfaces structurées (5a) sont disposées avec un décalage de 90°, **en ce que** la tige (5) présente entre les deux surfaces structurées (5a) un deuxième côté de guidage (5b) s'étendant dans la direction d'extension (5e) de la tige (5), et **en ce que** le deuxième élément de serrage (4) présente un ergot de guidage (4g), qui est agencé de telle manière que celui-ci soit dirigé vers le deuxième côté de guidage (5b).

2. Dispositif selon la revendication 1, **caractérisé en ce que** l'ergot de guidage (4g) est appliqué sur le deuxième côté de guidage (5b).

3. Dispositif selon la revendication 1 ou 2, **caractérisé en ce que** la surface structurée (5a) avec des dents s'étend le long d'une face plane.

4. Dispositif selon l'une quelconque des revendications 1 à 3, **caractérisé en ce que** le deuxième élément de serrage (4) présente dans la direction d'extension (5e) de la tige (5) une hauteur de construction (H) et **en ce que** la pièce d'encliquetage (4f) est configurée en s'étendant à l'intérieur de cette hauteur de construction (H), et s'engage à l'intérieur de cette hauteur de construction (H) dans la surface structurée (5a) de la tige (5).

5. Dispositif selon la revendication 4, **caractérisé en ce que** la face de guidage (4e) présente dans la direction d'extension (5e) de la tige (5) la hauteur de construction (H).

6. Dispositif selon l'une quelconque des revendications précédentes, **caractérisé en ce que** la partie d'extrémité (5d) de la tige (5) présente une partie d'encliquetage (5f), et **en ce que** la pièce de maintien (7a) présente une pièce d'encliquetage (7i) tournée vers la partie d'encliquetage (5f), qui s'engage dans la partie d'encliquetage (5f) de telle manière que la tige (5) soit fixée par rapport à la pièce de maintien (7a).

7. Dispositif selon la revendication 6, **caractérisé en ce que** la partie d'encliquetage (5f) présente une multiplicité de dents se succédant dans la direction d'extension (5e) de la tige (5), et **en ce que** la pièce d'encliquetage (7i) est configurée de façon élastique transversalement à la direction d'extension (5e) et présente une face frontale (7q) pour l'engagement dans les dents.

8. Dispositif selon la revendication 6 ou 7, **caractérisé en ce que** la pièce de maintien (7a) comprend sur le côté tourné vers la pièce de poussée (7b) une pièce de guidage (7m) avec un passage (7o), dans lequel le passage (7o) est configuré de telle manière que la tige (5) s'étende à travers le passage (7o), et dans lequel le passage (7o) est configuré au moins partiellement de façon adaptée à l'allure de la section transversale de la tige (5), de telle manière que la tige (5) puisse s'appliquer au moins partiellement sur le passage (7o).

9. Dispositif selon l'une quelconque des revendications 6 à 8, **caractérisé en ce que** la pièce de maintien (7a) est reliée à la pièce de liaison flexible (7c) par un bras de maintien (7k), et **en ce que** le bras de maintien (7k) comprend une face de poussée (71) dirigée vers la tige (5), sur laquelle la face frontale (5g) de la partie d'extrémité (5d) de la tige (5) peut s'appliquer.

10. Dispositif selon l'une quelconque des revendications précédentes, **caractérisé en ce que** l'applicateur (7) est réalisé en une seule pièce.
